# EUROPEAN PATENT APPLICATION

(11) **EP 4 151 725 A1**
(43) Date of publication of application: **22.03.2023**
(21) Application number: 21888679.4
(22) Date of filing: 08.11.2021
(51) Int. Cl.: C12N 5/10, C12N 15/861, A61K 39/00, A61K 48/00, A61P 31/00

(54) **METHOD FOR PREPARING ADENOVIRUS VECTOR VACCINE BY MEANS OF PERFUSION CULTURE PROCESS**

(30) Priority: 09.11.2020 CN 202011235524
(71) Applicant: Cansino Biologics Inc., Tianjin 300457 (CN)
(72) Inventor: XIAO, Meng, Tianjin 300457 (CN); LIU, Yunjie, Tianjin 300457 (CN); ZHU, Tao, Tianjin 300457 (CN); XU, Yunli, Tianjin 300457 (CN); XU, Can, Tianjin 300457 (CN); LI, Junqiang, Tianjin 300457 (CN); CHAO, Shoubai, Tianjin 300457 (CN)
(74) Representative: HGF
(86) International application number: PCT/CN2021/129182
(87) International publication number: WO 2022/095987

(57) **Abstract**

Provided is a method for preparing an adenovirus vector vaccine by means of a perfusion culture process. The method comprises a step of culturing adenovirus host cells, and in particular a step of adjusting the perfusion rate by means of at least two stages according to cell density. The method increases the single cell yield of a virus after infection and the specific activity of a virus harvest liquid while achieving high-density growth of adenovirus host cells.

## Description

### TECHNICAL FIELD

The present invention relates to the technical field of biological products, particularly to a method for culturing an adenovirus host cell, a method for producing an adenovirus and a method for preparing an adenovirus vector vaccine, and more particularly to a method for preparing an adenovirus vector vaccine by means of a perfusion culture process.

### BACKGROUND

Adenovirus is a non-enveloped DNA virus, and has the characteristics of susceptibility to infection, wide host range, low toxicity, safe use, large capacity, non-integration, low pathogenicity to humans, no carcinogenesis and the like, so that the adenovirus vector has become one of the most promising gene vectors for gene transfer.

The modified adenovirus removes an E1/E3 gene expression cassette, preventing the transcription of functional proteins which are dependent on the E1 region and the E3 region and the subsequent replication of virus DNA, as well as the production of virus coat protein, and the E1/E3-deleted adenovirus proliferates in cells capable of providing the E1/E3 region genes and proteins.

The adenovirus has the characteristics of wide host range and high transgene expression ability. At present, up to 300 kinds of human therapeutic or prophylactic recombinant adenovirus medicaments are used in clinical trials, and the widely used adenovirus vectors are human adenovirus serotypes Ad5 and Ad26 and chimpanzee adenovirus types 3 and 68. There are also several vaccines using adenovirus as a vector in clinical research.

The recombinant adenovirus vector vaccine uses adenovirus as a vector to recombine protective antigen genes into adenovirus genomes. After the adenovirus infects cells, the adenovirus genomes can be injected into the host cells together with the protective antigen genes, so that the protective antigens are expressed in the cells, and humoral and cellular immunities are produced. At present, there are several adenovirus vector vaccines in preclinical or clinical study, such as human immunodeficiency virus (HIV), rabies virus (RV), hepatitis B virus (HBV), hepatitis C virus (HCV), Dengue virus (DEN), herpes virus (EB), Ebola virus, and novel coronavirus.

293 cells are the most commonly used host cells for packaging and production of adenovirus vectors. The 293 cells are human renal epithelial cell lines, and there are a variety of derived strains, such as HEK293, 293T/17, etc. The 293 cells are generally adherent to walls for growth, but there are also cell lines adapted to suspension culture. The HEK293 cells comprise the E1/E3 region of the adenovirus, and thus, they can be used for producing replication-defective adenovirus vectors.

In the prior art, the production of adenovirus by the 293 cells is limited by low cell density and low virus production in the cells, resulting in low yield. The main reason for low cell density is that the cell culture is affected by nutrient supply, oxygen and metabolism, making it difficult to culture the 293 cells at high density. The competition between infected cells and normal cells in growth and nutrients, and the state of infected cells lead to low virus production. Although regular supplementation of fresh medium, which is used to maintain a nutrient environment and can improve cell growth, such growth is limited.

### SUMMARY

To overcome the defects of the prior art, the present invention provides a method for culturing an adenovirus host cell, which comprises a step of perfusion culture, and in particular a step of adjusting the perfusion rate (e.g., adjusting the perfusion rate by means of at least two stages) according to cell density.

Specifically, the method described above comprises the following steps:
(1) inoculating host cells for cell culture;
(2) starting perfusion at a perfusion rate of 1-3 VVD (container volume/day) (e.g., 1 VVD, 1.5 VVD, 2 VVD, 2.5 VVD or 3 VVD) after a cell density reaches 1 × 10⁶-5 × 10⁶ cells/mL (specifically, 1 × 10⁶ cells/mL, 2 × 10⁶ cells/mL, 3 × 10⁶ cells/mL, 4 × 10⁶ cells/mL or 5 × 10⁶ cells/mL);
(3) adjusting the perfusion rate to 2-4 VVD (e.g. 2 VVD, 2.5 VVD, 3 VVD, 3.5 VVD or 4 VVD) after the cell density grows to 5 × 10⁶-10 × 10⁶ cells/mL (specifically, 5 × 10⁶ cells/mL, 6 × 10⁶ cells/mL, 7 × 10⁶ cells/mL, 8 × 10⁶ cells/mL, 9 × 10⁶ cells/mL or 10 × 10⁶ cells/mL).

Specifically, the cell culture described above is performed in a bioreactor (e.g., a single-use stirred reactor or torrent reactor).

Specifically, the perfusion described above is performed by using a continuous perfusion device; and more specifically, the continuous perfusion device uses an alternating tangential flow filtration system, wherein a filtration pore size of a hollow fiber column is 0.1-0.8 µm (specifically, 0.1 µm, 0.2 µm, 0.4 µm, 0.6 µm or 0.8 µm). Specifically, a liquid exchange rate in the perfusion described above can be 1-12 L/h (specifically, 1 L/h, 2 L/h, 3 L/h, 4 L/h, 5 L/h, 6 L/h, 7 L/h, 8 L/h, 9 L/h, 10 L/h, 11 L/h or 12 L/h).

Specifically, a temperature in the cell culture described above can be 32-38°C(specifically, 32°C, 33°C, 34°C, 35°C, 36°C, 37°C or 38°C).

Specifically, a pH in the cell culture described above can be 6.5-7.5 (specifically, 6.5, 6.6, 6.8, 7.0, 7.2, 7.4 or 7.5).

Specifically, a concentration of dissolved oxygen in the cell culture described above can be 30%-80% (specifically, 30%, 40%, 50%, 60%, 70% or 80%).

Specifically, a concentration of CO₂ in the cell culture described above can be 4%-8% (specifically, 4%, 5%, 6%, 7% or 8%).

Specifically, a stirring velocity in the cell culture described above can be 30-200 rpm (specifically, 30 rpm, 40 rpm, 50 rpm, 60 rpm, 80 rpm, 100 rpm, 120 rpm, 140 rpm, 160 rpm, 180 rpm or 200 rpm).

Specifically, the medium used in the cell culture described above can be any medium known to be suitable for the growth of host cells.

Specifically, the method described above further comprises a step of continuously tracking glutamine consumption and performing supplementation during the process of cell culture.

Specifically, a concentration of glutamine in the cell culture described above is maintained at 2 mM or more (specifically, 2 mM, 3 mM, 4 mM, 5 mM, 6 mM, 7 mM, 8 mM, 9 mM, 10 mM, 12 mM, 14 mM, 16 mM, 18 mM, 20 mM or 30 mM). Specifically, the method described above may further comprise a step of performing scale-up culture for the host cells prior to the step (1).

Specifically, a passage density in the scale-up culture described above is 1 × 10⁶-4 × 10⁶ cells/mL (specifically, 1 × 10⁶ cells/mL, 2 × 10⁶ cells/mL, 3 × 10⁶ cells/mL or 4 × 10⁶ cells/mL).

Specifically, a passage time in the scale-up culture described above is 48-90 h (specifically, 48 h, 60 h, 72 h, 84 h or 90 h).

Specifically, a temperature in the scale-up culture described above can be 32-38°C (specifically, 32°C, 33°C, 34°C, 35°C, 36°C, 37°C or 38°C).

Specifically, a concentration of CO₂ in the cell culture described above can be 4%-8% (specifically, 4%, 5%, 6%, 7% or 8%).

Specifically, a stirring velocity in the cell culture described above can be 100-170 rpm (specifically, 100 rpm, 110 rpm, 120 rpm, 130 rpm, 140 rpm, 150 rpm, 160 rpm or 170 rpm).

Specifically, the host cell described above is a cell capable of packaging the adenovirus.

Specifically, the host cell described above can grow in adherent culture or in suspension culture, and particularly those host cells adapted to suspension culture. In one embodiment of the present invention, the host cell described above is a 293 cell, such as, but not limited to, an HEK293 cell, an HEK293.CS cell, etc. Specifically, the adenovirus described above may be replication-competent or replication-defective, and particularly replication-defective adenovirus.

Specifically, the adenovirus described above may be a human adenovirus (e.g., human adenovirus type 5 (AdHu5), AdHu4, AdHu7, AdHu11, AdHu26, AdHu55, etc.), an animal adenovirus vector such as a chimpanzee adenovirus vector (e.g., chimpanzee adenovirus type 68 (AdC68), AdC3, etc.); in one embodiment of the present invention, the adenovirus described above is AdHu5.

In one embodiment of the present invention, the adenovirus described above is a recombinant adenovirus comprising a coding exogenous gene, e.g., for gene therapy, for vaccines, or the like.

Specifically, the adenovirus described above comprises a gene (full-length or partial sequence) of a structural protein (e.g., one or more of the followings: S protein, M protein, E protein , N protein) of SARS-CoV-2, and particularly the gene of the S protein.

In one embodiment of the present invention, the adenovirus described above is AdHu5 comprising the gene of the S protein of SARS-CoV-2.

The present invention further provides a method for producing an adenovirus, which comprises the following steps:
(a) culturing an adenovirus host cell;
(b) inoculating virus for culture.

Specifically, the step (a) described above may be the step in the method for culturing the adenovirus host cell described above in the present invention.

In one embodiment of the present invention, the method for producing an adenovirus described above comprises the following steps:
(1) inoculating host cells for cell culture;
(2) starting perfusion at a perfusion rate of 1-3 VVD (e.g., 1 VVD, 1.5 VVD, 2 VVD, 2.5 VVD or 3 VVD) after a cell density reaches 1 × 10⁶-5 × 10⁶ cells/mL (specifically, 1 × 10⁶ cells/mL, 2 × 10⁶ cells/mL, 3 × 10⁶ cells/mL, 4 × 10⁶ cells/mL or 5 × 10⁶ cells/mL);
(3) adjusting the perfusion rate to 2-4 VVD (e.g. 2 VVD, 2.5 VVD, 3 VVD, 3.5 VVD or 4 VVD) after the cell density grows to 5 × 10⁶-10 × 10⁶ cells/mL (specifically, 5 × 10⁶ cells/mL, 6 × 10⁶ cells/mL, 7 × 10⁶ cells/mL, 8 × 10⁶ cells/mL, 9 × 10⁶ cells/mL or 10 × 10⁶ cells/mL);
(4) inoculating virus for culture.

Specifically, the virus inoculation described above is performed after the density of the host cells in the step (3) reaches 10 × 10⁶-50 × 10⁶ cells/mL (specifically, 10 × 10⁶ cells/mL, 20 × 10⁶ cells/mL, 30 × 10⁶ cells/mL, 40 × 10⁶ cells/mL or 50 × 10⁶ cells/mL).

Specifically, in the step (4) described above, the culture method includes: perfusion culture, batch culture, fed-batch culture and the like, and particularly perfusion culture; more specifically, the perfusion rate may be 1-3 VVD (specifically, 1 VVD, 1.5 VVD, 2 VVD, 2.5 VVD or 3 VVD).

Specifically, in the step (4) described above, the medium used in the culture can be any medium known to be suitable for the amplification and production of adenovirus, such as CD293 medium, SFM4HEK293 medium, Ex-Cell293 medium and the like, and the medium used in the examples of the present invention can be any medium described above.

Specifically, the other culture conditions in the step (4) described above have the definitions of the corresponding conditions in the method for culturing the adenovirus host cell described above.

Specifically, the method for producing the adenovirus described above may further comprise step (5): harvesting the virus.

Specifically, the adenovirus described above may be replication-competent or replication-defective, and particularly replication-defective adenovirus.

Specifically, the adenovirus described above may be a human adenovirus (e.g., human adenovirus type 5 (AdHu5), AdHu4, AdHu7, AdHu11, AdHu26, AdHu55, etc.), an animal adenovirus vector such as a chimpanzee adenovirus vector (e.g., chimpanzee adenovirus type 68 (AdC68), AdC3, etc.); in one embodiment of the present invention, the adenovirus described above is AdHu5.

In one embodiment of the present invention, the adenovirus described above is a recombinant adenovirus comprising a coding exogenous gene, e.g., for gene therapy, for vaccines, or the like.

Specifically, the adenovirus described above comprises a gene (full-length or partial sequence) of a structural protein (e.g., one or more of the followings: S protein, M protein, E protein, N protein) of SARS-CoV-2, and particularly the gene of the S protein.

In one embodiment of the present invention, the adenovirus described above is AdHu5 comprising the gene of the S protein of SARS-CoV-2.

The present invention further provides a method for preparing an adenovirus vector vaccine, which comprises the steps of the method for culturing the adenovirus host cell or the method for producing the adenovirus described above.

Specifically, the adenovirus vector described above comprises a gene (full-length or partial sequence) of a structural protein (e.g., one or more of the followings: S protein, M protein, E protein, N protein) of SARS-CoV-2, and particularly the gene of the S protein.

In one embodiment of the present invention, the adenovirus vector described above is AdHu5 adenovirus vector comprising the gene of the S protein of SARS-CoV-2. Specifically, the method for preparing the adenovirus vector vaccine described above can further comprise a step of preparing the adenovirus into a suitable formulation. In one embodiment of the present invention, the formulation described above is an injection.

In another embodiment of the present invention, the formulation described above is a formulation for mucosal administration, for example, a nasal drop, an aerosol, a spray, a powder, a gel, a microsphere, a liposome, a membrane, a suspension, etc.

The present invention has the following beneficial effects:
1. The technical scheme provided by the present invention is a technical method for high-density culture of adenovirus host cells (such as 293 cells) by means of a perfusion culture process. The selected culture conditions and perfusion conditions are suitable for the growth of the adenovirus host cells (such as 293 cells), and higher cell density and virus infection than those in batch and conventional perfusion processes can be obtained by the technical method. A fresh medium can be continuously supplemented into the reactor through the perfusion culture and filtration device, so that metabolic waste is taken away in real time, the high-density culture of cells is achieved, and the virus titer is significantly improved after the inoculation of adenovirus;
2. The technical scheme provided by the present invention can culture cells to high density and obtain high-titer virus harvest liquid, and the upstream perfusion culture liquid is purified by a method combining lysis, clarification and column chromatography, so that a variety of impurities are effectively removed, and a high-purity stock solution is obtained; and
3. The technical scheme provided by the present invention increases the single cell yield of a virus after infection and the specific activity of a virus harvest liquid while achieving high-density growth of adenovirus host cells (e.g., 293 cells).

### BRIEF DESCRIPTION OF THE DRAWINGS

FIG. 1 is a graph showing the comparison between the highest density of 293 cells in perfusion culture and that in batch culture.
FIG. 2 is a graph showing the comparison between a specific activity of adenovirus stock solution in perfusion culture process and that in batch culture process.

### DETAILED DESCRIPTION

Unless otherwise defined, all scientific and technical terms used herein have the same meaning as commonly understood by those skilled in the art to which the present invention relates.

Genes of SARS-CoV-2 and its structural proteins can be searched by techniques known in the art, for example, the genes of SARS-CoV-2 can be shown in GenBank: MT419849.1, and its structural proteins: the genes of S protein, E protein, M protein can be shown in 21387-25208, 26069-26296, 26347-27015 of GenBank: MT419849.1.

The disclosures of the various publications, patents, and published patent specifications cited herein are hereby incorporated by reference in their entirety.

The technical schemes of the present invention will be clearly and completely described below with reference to the examples of the present invention, and it is obvious that the described examples are only a part of the examples of the present invention but not all of them. Based on the examples of the present invention, all other examples obtained by those of ordinary skill in the art without creative work shall fall within the protection scope of the present invention.

### Example 1: Determination of Process Parameters for 293 Cells Cultured by Perfusion Method

Perfusion process 1: 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. Perfusion process 2: 293 cells were resuscitated, amplified and then inoculated into a fermenter, and the perfusion was started after the cell density reached 1 × 10⁶ cells/mL or 5 × 10⁶ cell/mL. The perfusion rate was maintained constant at 1 VVD or 3 VVD throughout the culture.

Perfusion process 3: 293 cells were resuscitated, amplified and then inoculated into a fermenter, and the perfusion was started after the cell density reached 1 × 10⁶ cells/mL or 5 × 10⁶ cell/mL. The perfusion rate was maintained constant at 2 VVD or 4 VVD throughout the culture.

In the above 3 processes, the reactor parameters were as follows: the culture temperature was 37 °C, the pH value was adjustable from 6.5 to 7.5, the concentration of dissolved oxygen was 30%-80%, and the stirring velocity was adjustable from 30 rpm to 40 rpm.

The density and viability of 293 cells cultured by different perfusion processes were measured. The results are shown in Tables 1 to 3.

**Table 1. The density and viability of 293 cells cultured by perfusion process (perfusion process 1)**

| Density of inoculation | 1 × 10⁶ cells/mL | Notes |
|---|---|---|
| Perfusion velocity in the first stage | 2VVD | |
| Perfusion velocity in the second stage | 3VVD | Started when the cells grew to 5 × 10⁶ cells/mL |
| Density | 11 × 10⁷ cells/mL | |
| Viability | 98% | |

The results showed that the perfusion was started at the perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. The highest growth density of the cells could reach 11 × 10⁷ cells/mL.

**Table 2. The density and viability of 293 cells cultured by perfusion process (perfusion process 2)**

| | 1 × 10⁶ cells/mL | | 5 × 10⁶ cells/mL | |
|---|---|---|---|---|
| | 1VVD | 3VVD | 1VVD | 3VVD |
| Density | 65 × 10⁶ cells/mL | 70 × 10⁶ cells/mL | 50 × 10⁶ cells/mL | 60 × 10⁶ cells/mL |
| Viability | 94% | 96% | 92% | 93% |

The results showed that the perfusion was started after the cell density reached 1 × 10⁶ cells/mL or 5 × 10⁶ cells/mL, and the perfusion rate was maintained constant at 1 VVD or 3 VVD throughout the culture. The highest growth density of the cells could reach 7 × 10⁷ cells/mL.

**Table 3. The density and viability of 293 cells cultured by perfusion process (perfusion process 3)**

| | 1 × 10⁶ cells/mL | | 5 × 10⁶ cells/mL | |
|---|---|---|---|---|
| | 2VVD | 4VVD | 2VVD | 4VVD |
| Density | 85 × 10⁶ cells/mL | 80 × 10⁶ cells/mL | 70 × 10⁶ cells/mL | 80 × 10⁶ cells/mL |
| Viability | 96% | 96% | 93% | 94% |

The results showed that the perfusion was started after the cell density reached 1 × 10⁶ cells/mL or 5 × 10⁶ cells/mL , and the perfusion rate was maintained constant at 2 VVD or 4 VVD throughout the culture. The highest growth density of the cells could reach 70 × 10⁶ cells/mL.

Based on the results above, higher cell density could be obtained by using the perfusion process 1, and the cell viability was also higher than that in the processes 2 and 3.

### Example 2: Comparisons Between Cell Density and Viability in Perfusion Culture Process and that in Batch Culture Process

Test 1: the culture was performed by using conventional batch culture process in the art.

Test 2: the perfusion process 1 in the Example 1 was used, i.e., 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL.

The other parameters (such as culture temperature, pH, concentration of dissolved oxygen and stirring velocity) in the culture process of Tests 1 and 2 were substantially the same, and the cell density and viability after the culture were measured. The results are shown in Table 4.

**Table 4. The viability and density of 293 cells cultured by batch process and perfusion process**

| | Batch process-Test 1 | Perfusion process-Test 2 |
|---|---|---|
| Density | 3.8 × 10⁶ cells/mL | 13 × 10⁷ cells/mL |
| Viability | 96% | 98% |

The results showed that higher cell density could be obtained by using the perfusion process, and the cell density and viability were higher than those in the batch process.

### Example 3: Effect of Glutamine on Cell Production in Perfusion Process

Test 1: 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. The concentration of glutamine was not supplemented in the perfusion process.

Test 2: 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. The concentration of glutamine in the perfusion process was monitored, and glutamine was supplemented to maintain the concentration of glutamine at 2 mM.

Test 3: 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. The concentration of glutamine in the perfusion process was monitored, and glutamine was supplemented to maintain the concentration of glutamine at 10 mM.

Test 4: 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. The concentration of glutamine in the perfusion process was monitored, and glutamine was supplemented to maintain the concentration of glutamine at 20 mM.

In the above Tests 1 to 4, the reactor parameters were as follows: the culture temperature was 37 °C, the pH value was adjustable from 6.5 to 7.5, the concentration of dissolved oxygen was adjustable from 30% to 80%, and the stirring velocity was adjustable from 30 rpm to 40 rpm.

The highest cell density and viability after the end of the culture were measured. The results are shown in Table 5.

**Table 5. The density and viability of 293 cells cultured by different perfusion processes**

| | Test 1 | Test 2 | Test 3 | Test 4 |
|---|---|---|---|---|
| Concentration of glutamine | 0 | 2 mM | 10 mM | 20 mM |
| Density | 11 × 10⁷ cells/mL | 29 × 10⁷ cells/mL | 30 × 10⁷ cells/mL | 30 × 10⁷ cells/mL |
| Viability | 98% | 99% | 99% | 99% |

The results showed that higher cell density could be obtained by using the perfusion process with the supplementation of glutamine concentration, and the cell density and viability were higher than those in the process without the supplementation of glutamine.

### Example 4: Comparison Between the Highest Density of 293 Cells in Perfusion Culture and that in Batch Culture

Test 1: the culture was performed by using conventional batch culture process in the art.

Test 2: the perfusion culture process of the perfusion process test 2 in Example 3 was used, i.e., 293 cells were resuscitated, amplified and then inoculated into a fermenter, and the perfusion was started at a perfusion rate of 2 VVD after the cell density reached 1 × 10⁶ cells/mL. The cells were continuously cultured, and the perfusion rate was adjusted to 3 VVD after the cell density grew to 5 × 10⁶ cells/mL. The concentration of glutamine in the perfusion process was monitored, and glutamine was supplemented to maintain the concentration of glutamine at 2 mM.

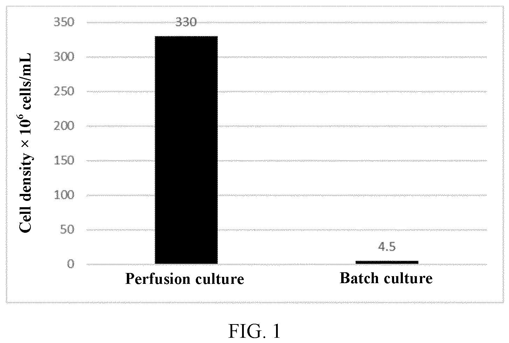

The highest cell density and viability after the end of the culture were measured. The results are shown in FIG. 1.

The results in FIG. 1 showed that the highest culture density in the batch culture reached 3.8 × 10⁶ cells/mL. In contrast, in the perfusion culture, a fresh medium could be continuously supplemented into the reactor through the continuous perfusion device to replace cell metabolic waste within the reactor and maintain sufficient nutrients for the cells. The cell density cultured by the perfusion process could reach up to 33 × 10⁷ cells/mL, which was about 100 times higher than that of the cells cultured by the batch process. Therefore, the perfusion culture has significant advantages.

### Example 5: Virus Yield in Perfusion Culture Process

Test 1: a perfusion process: perfusion culturing was performed until the cell density reached 10 × 10⁶ cells/mL, recombinant novel coronavirus (adenovirus vector) strains (prepared according to the known prior art) were inoculated, the glutamine concentration in the medium was monitored and then supplemented to 3 mM or more, and the perfusion rate of 1 VVD or 3 VVD was selected. After the completion of culture, the titer of the virus harvest liquid was determined by enzyme-linked immunosorbent assay.

Test 2: a batch process: viruses were inoculated for batch culture, glutamine was not supplemented, and the perfusion was not performed after the inoculation of viruses. Other parameters were substantially the same during the culture in Tests 1 to 2. The results are shown in Table 4.

**Table 6. Comparison between virus production of 293 cells in perfusion culture and that in batch culture**

| | Perfusion culture | | Batch culture |
|---|---|---|---|
| | 1VVD | 3VVD | N/A |
| Cell density/viability when inoculating viruses | 11.2 × 10⁶ cells/mL 94% | 15.2 × 10⁶ cells/mL 97% | 0.9 × 10⁶ cells/mL 94% |
| Titer of virus harvest liquid | 1.364×10¹⁰ IFU/mL | 1.7×10¹⁰ IFU/mL | 1.1×10⁸ IFU/mL |
| Total virus amount | 8.6×10¹⁴ IFU | 7.6×10¹⁴ IFU | 5.5×10¹²IFU |
| Single cell yield | 1218 IFU/cell | 1118 IFU/cell | 122 IFU/cell |

Comparing the virus production of single cell in the perfusion culture and that in the batch culture, higher yield of the single cell could be obtained by using the perfusion culture method, which was increased by about 10 times, and the production capacity in single tank was increased by nearly 100 times.

### Example 6: Study on Perfusion Culture Process with Higher Virus Specific Activity

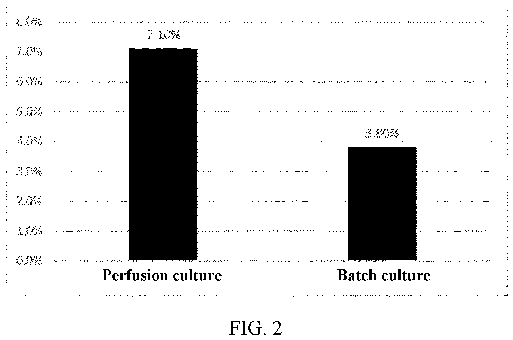

The adenovirus harvest liquids in perfusion culture and in batch culture were purified, respectively. The titer and the number of virus particles were measured, and the specific activities were calculated. The results are shown in FIG. 2.

The results showed that the specific activity of the adenovirus stock solution obtained by batch culture was 3.8%, and the specific activity of the adenovirus stock solution obtained by perfusion culture was 7.1%. Therefore, more live virus particles could be obtained by perfusion culture of adenovirus, which was superior to that obtained by batch culture.

The above description is only for the purpose of illustrating the preferred example of the present invention, and is not intended to limit the scope of the present invention. Any modifications, equivalents and the like made without departing from the spirit and principle of the present invention should be included in the protection scope of the present invention.

The foregoing examples and methods described herein may vary based on the abilities, experience, and preferences of those skilled in the art.

The certain order in which the steps of the method are listed in the present invention does not constitute any limitation on the order of the steps of the method.

## Claims

1. A method for culturing an adenovirus host cell, comprising the following steps:
(1) inoculating host cells for cell culture;
(2) starting perfusion at a perfusion rate of 1-3 VVD after a cell density reaches 1 × 10⁶-5 × 10⁶ cells/mL;
(3) adjusting the perfusion rate to 2-4 VVD after the cell density grows to 5 × 10⁶-10 × 10⁶ cells/mL.

2. The method according to claim 1, wherein the perfusion is performed by using a continuous perfusion device; the continuous perfusion device uses an alternating tangential flow filtration system, wherein a filtration pore size of a hollow fiber column is 0.1-0.8 µm.

3. The method according to claim 1, wherein a concentration of glutamine is maintained at 2 mM or more in the cell culture.

4. The method according to any one of claims 1 to 3, wherein the host cell is a 293 cell.

5. The method according to claim 4, wherein the host cell is an HEK293 cell or an HEK293.CS cell.

6. A method for producing an adenovirus, comprising the following steps:
(1) inoculating host cells for cell culture;
(2) starting perfusion at a perfusion rate of 1-3 VVD after a cell density reaches 1 × 10⁶-5 × 10⁶ cells/mL;
(3) adjusting the perfusion rate to 2-4 VVD after the cell density grows to 5 × 10⁶-10 × 10⁶ cells/mL;
(4) inoculating virus for culture.

7. The method according to claim 6, wherein in the step (4), the culture is a perfusion culture at a perfusion rate of 1-3 VVD.

8. The method according to claim 6 or 7, wherein the perfusion is performed by using a continuous perfusion device; the continuous perfusion device uses an alternating tangential flow filtration system, wherein a filtration pore size of a hollow fiber column is 0.1-0.8 µm.

9. The method according to claim 6, wherein a concentration of glutamine is maintained at 2 mM or more in the culture.

10. The method according to claim 6, wherein the host cell is a 293 cell.

11. The method according to claim 10, wherein the host cell is an HEK293 cell or an HEK293.CS cell.

12. The method according to claim 6, wherein the adenovirus is a human adenovirus or a chimpanzee adenovirus.

13. The method according to claim 6, wherein the adenovirus is selected from: AdHu5, AdHu4, AdHu7, AdHu11, AdHu26, AdHu55, AdC68, AdC3.

14. The method according to claim 6, wherein the adenovirus is a recombinant adenovirus comprising a coding exogenous gene.

15. The method according to claim 14, wherein the adenovirus comprises a gene of a structural protein of SARS-CoV-2, the structural protein is selected from: one or more of the followings: S protein, M protein, E protein, N protein.

16. A method for preparing an adenovirus vector vaccine, comprising the steps in the method according to any one of claims 1 to 15.
